# EUROPEAN PATENT APPLICATION

(11) **EP 3 312 274 A1**
(43) Date of publication of application: **25.04.2018**
(21) Application number: 16741971.2
(22) Date of filing: 17.06.2016
(51) Int. Cl.: C12N 9/22, C12N 15/62, C12N 15/90

(54) **NUCLEIC ACID MOLECULE, FUSION PROTEIN AND METHOD FOR ALTERING THE GENETIC MATERIAL OF A CELL**

(30) Priority: 19.06.2015 ES 201530874
(71) Applicant: Biopraxis Research AIE, 01510 Miñano (Alava) (ES); Consejo Superior De Investigaciones Científicas, 28006 Madrid (ES); Fundació d'Investigació Sanitària de Les Illes Balears, 07120 Palma (ES); Universidad Del Pais Vasco Euskal Herriko Unibertsitatea, 48940 Leioa (Vizcaya) (ES); Universidad de Barcelona, 08907 Hospitalet (ES)
(72) Inventor: GAINZA LAFUENTE, Eusebio Jesús, 01510 Miñano (ES); GAINZA LUCEA, Garazi, 01510 Miñano (ES); DEL POZO PEREZ, Angel, 01510 Miñano (ES); PASTOR NAVARRO, Marta, 01510 Miñano (ES); PEDRAZ MUÑOZ, José Luis, 48940 Leioa (ES); VIÑAS CIORDIA, Miguel, 08907 Hospitalet (ES); BACHILLER PEREZ, Daniel, 07110 Bunyola (ES); GALVEZ JEREZ, Victor, 07120 Palma (ES)
(74) Representative: Igartua, Ismael
(86) International application number: PCT/ES2016/070459
(87) International publication number: WO 2016/203088

(57) **Abstract**

The invention relates to a nucleic acid molecule encoding a fusion protein, the nucleic acid molecule comprising in the 5'---3' transcription direction at least one nucleic acid sequence encoding a DNA binding domain, and one nucleic acid sequence encoding a Fokl-type catalytic domain, wherein at its 3' end it comprises a nucleic acid sequence encoding a peptide comprising between 18 and 23 amino acids, to a fusion protein that can be obtained, for example, from the transcription of said molecule, and to a method for modifying the genetic material of a cell by means of the use of said molecule or protein.

## Description

### TECHNICAL FIELD

The present invention relates to molecular tools that allow modifying the genetic material of a cell.

### PRIOR ART

New types of molecular tools that allow modifying the genetic material of cells have appeared in the last few years. By way of example, tools such as Zinc Finger nucleases, CRISPR/Cas9 or TALENs have marked a technological revolution in this field since they are all capable of cutting the DNA double helix in specific genome sequences, which opens up the real possibility of modifying the genetic information of live cells at will. These tools, also known as sequence-specific nucleases, are characterized by having one DNA binding domain and another catalytic domain that cuts at a sequence adjacent to the sequence recognized by the binding domain.

Many attempts have been made to improve the efficacy and specificity of said tools in targeted genome editing techniques. By way of example, EP2510096 A1 discloses a TALEN improving cutting selectivity, and WO2012015938 A2 discloses new Fokl-type domain variants with at least one mutation in one of the amino acid residues with respect to the wild type.

The modification of a peptide sequence by means of adding a sequence encoding between 18 and 23 amino acids creates a different protein that can be inert or have functional characteristics other than the starting proteins. In fact, cases have been described in which the addition of 2A peptides at the C-terminal end of a protein brought about its inactivation, such as, for example, one of the structures described in Anal Biochem, 2005.343 (1): p.116-24 "FDMV-2A sequence and protein arrangement contribute to functionality of CYP2B1-reporter fusion protein".

### DISCLOSURE OF THE INVENTION

The object of the invention is to provide a nucleic acid molecule, a fusion protein and a method for modifying the genetic material of a cell, as defined in the claims.

One aspect of the invention relates to a nucleic acid molecule encoding a fusion protein, the nucleic acid molecule comprising in the 5'---3' transcription direction at least:
(a) one nucleic acid sequence encoding a DNA binding domain; and
(b) one nucleic acid sequence encoding a Fokl-type catalytic domain, wherein at its 3' end it comprises a nucleic acid sequence encoding a peptide comprising between 18 and 23 amino acids.

Another aspect of the invention relates to a fusion protein comprising in the N-terminus to C-terminus direction at least one DNA binding domain and at least one Fokl-type catalytic domain, wherein said Fokl-type catalytic domain has a peptide comprising between 18 and 23 amino acids bound to its C-terminal end.

Another aspect of the invention relates to a method for modifying the genetic material of a cell, comprising the steps of:
(i) providing a cell containing a target DNA nucleotide sequence; and
(ii) introducing in said cell at least said nucleic acid molecule, or at least one nucleic acid molecule encoding at least said fusion protein, and inducing expression of said at least one nucleic acid molecule; or
(iii) introducing in said cell at least one said fusion protein such that the binding domain recognizes the target nucleotide sequence and the Fokl-type catalytic domain can cut at a nucleotide sequence adjacent to the target nucleotide sequence.

The presence of a new element at the C-terminal end of the Fokl domain entails about a 20% increase in the size of said domain. Studies conducted by the inventors have demonstrated that this modification improves Fokl cutting efficacy.

The nucleic acid molecule or the fusion protein of the invention can be useful in the treatment of genetic disorders, particularly monogenic genetic disorders. Therefore, another aspect of the invention relates to the nucleic acid molecule or the protein defined above or the cell modified according to the method defined above, for use as a drug or a therapeutic composition.

Another aspect of the invention relates to a method for the treatment and/or prevention of a disease, preferably an immune system disease, preferably caused by HIV and/or similar species, or of a monogenic genetic disorder, and which comprises administering a therapeutically effective amount of the nucleic acid molecule or the fusion protein defined above or of the cell modified according to the method defined above, together with pharmaceutically acceptable carriers or excipients, in a subject in need of said treatment and/or prevention, including a human.

These and other advantages and features of the invention will become evident in view of the drawings and detailed description of the invention.

### DESCRIPTION OF THE DRAWINGS

Figure 1 shows a diagram of a bicistronic gene comprising a ribonucleic acid molecule according to one embodiment of the invention.
Figure 2 shows a diagram of a tricistronic gene comprising a ribonucleic acid molecule according to one embodiment of the invention.
Figure 3 shows the structure of the proteins produced from the bicistronic gene of Figure 1.
Figure 4 shows the mechanism of action of the proteins produced from the bicistronic gene of Figure 1.
Figure 5 shows the results of Example 2 of the activity of a fusion protein of the invention with respect to the activity of a protein of the prior art.
Figure 6 shows a scatter plot of cells transfected with two fusion proteins according to an embodiment of Example 3.
Figure 7 shows the activity of the fusion proteins and selection by fluorescence according to an embodiment of Example 3.
Figure 8 shows the production of T cells with two null copies of the CCR5 gene according to an embodiment of Example 4..

### DETAILED DISCLOSURE OF THE INVENTION

A first aspect of the invention relates to the nucleic acid molecule encoding a fusion protein that was developed by the inventors and comprises in the 5'---3' transcription direction at least one nucleic acid sequence encoding a DNA binding domain and one nucleic acid sequence encoding a Fokl-type catalytic domain, wherein at the 3' end of said nucleic acid sequence encoding the Fokl-type catalytic domain it comprises a nucleic acid sequence encoding a peptide comprising between 18 and 23 amino acids, preferably 21 amino acids.

The terms nucleic acid, polynucleotide, oligonucleotide or nucleotide are interchangeable and refer to deoxyribonucleic acids or ribonucleic acid, in a linear or circular conformation, either single-stranded or double-stranded. These terms can include known analogs of natural nucleotides, as well as nucleotides that are modified in the base, sugar and/or phosphate moieties. Generally, an analog of a particular nucleotide has the same base pairing specificity as said particular nucleotide.

In the context of the present invention, fusion protein is understood as a protein comprising at least one polypeptide comprising a DNA binding or recognition domain and a Fokl-type catalytic domain cutting said DNA.

In the context of the invention, Fokl-type catalytic domain is understood as the catalytic domain of a Fokl enzyme. The Fokl enzyme is a type IIS restriction endonuclease of bacteria which is found in nature in *Flavobacterium okeanokoites.* In the context of the invention, the catalytic domain can be in its wild-type homodimer conformation or obligate heterodimer conformation.

In a particular embodiment, the peptide comprising between 18 and 23 amino acids is a 2A-type peptide.

In the context of the invention, the 2A-type peptide refers to a peptide sequence consisting of between 18 and 23 amino acids located between two functional polypeptides or proteins.

2A peptides are originally found in different viruses of the *Picornavirus* family, where they act in the protein synthesis process. All the proteins encoded by the genome of these viruses are comprised in a single polycistron or single reading frame. The 2A elements induce interruption of the polyprotein chain synthesis process by means of the process referred to as ribosomal skipping, whereby different proteins encoded in the genome of the virus end up being produced independently.

In a particular embodiment, said 2A-type peptide comprises 21 amino acids. Preferably, the 2A-type peptide is a T2A-type peptide.

In a particular embodiment, said 2A-type peptide comprises the amino acid sequence SEQ ID NO: 1.

DNA binding domain is understood as a domain comprised in a polypeptide, comprising at least one structure recognizing the DNA to which it is bound. A DNA binding domain can recognize a specific DNA sequence (a recognition sequence) or can have a general affinity for the DNA. Examples of polypeptides comprising the DNA binding domain are: ZFN, CRISPR/Cas9 or TALE.

In a particular embodiment, the binding domain of the nucleic acid molecule according to the preceding features is a TALE-type domain.

The term TALE is understood as TAL effector or TALE polypeptide or TALE protein comprising a DNA binding domain having a plurality of DNA binding repetitions, where each repetition comprises an RVD determining the recognition of a base of the target DNA.

In a particular embodiment, the nucleic acid molecule according to the preceding features comprises in the 5'---3' transcription direction:
(a) the nucleic acid sequence encoding the DNA binding domain comprising a first segment consisting of between 150 and 600 base pairs, preferably between 470 and 550 base pairs, encoding the N-terminal end of a TALE protein and a nuclear localization signal, a second segment consisting of between 1100 and 2900 base pairs encoding the TALE-type DNA binding domain, and a third segment consisting of between 40 and 850 base pairs encoding the C-terminal end of the TALE protein; and
(b) the nucleic acid sequence encoding the Fokl-type catalytic domain comprising a first segment consisting of between 550 and 600 base pairs, preferably between 590 and 600 base pairs, encoding the Fokl-type catalytic domain followed by a segment consisting of between 50 and 70 base pairs, preferably 69 base pairs, encoding the peptide comprising between 18 and 23 amino acids, preferably 21 amino acids.

In the context of the invention, nuclear localization signal (hereinafter NLS) is understood as an amino acid sequence labeling a protein so that it can be imported into the cell nucleus through transport proteins known as import receptors.

In a particular embodiment, the nucleic acid molecule according to the preceding features, in the 5'---3' transcription direction after the nucleic acid sequence encoding the peptide comprising between 18 and 23 amino acids, comprises a nucleic acid sequence encoding a protein identifying transcription.

In a preferred embodiment, after the nucleic acid sequence encoding the Fokl-type catalytic domain, the nucleic acid molecule comprises a nucleic acid sequence encoding the protein identifying transcription comprising between 700 and 1000 base pairs.

In the context of the invention, protein identifying transcription is understood as a label which, due to its transcription, allows identifying and selecting those cells in which the fusion protein of the invention has been expressed. Products conferring antibiotic resistance, products conferring a selective growth advantage when the nucleic acid molecule is expressed in the presence of a substrate and/or fluorescent proteins are included as labels, without limitation.

In a preferred embodiment, the protein identifying transcription is a fluorescent protein. Examples of fluorescent proteins include, without limitation, GFP, tdTomato, IRFP, mEmerald, DsRed, EBFP, EYFP, Cerulean, ECFP, etc. In a preferred embodiment, the fluorescent protein is mCherry protein or EGFP protein.

The nucleic acid molecule according to the preceding features can form a monocistronic gene or a polycistronic gene, preferably a bicistronic gene or a tricistronic gene.

One of the advantages of the nucleic acid molecule forming a polycistronic gene is that the fusion protein and the protein identifying transcription are expressed from one and the same promoter. Identification and selection by fluorescence, for example, allows obtaining a cell population rich in cells that have undergone a genetic modification process. The structure of the nucleic acid molecule of the invention minimizes the risk of selecting false positives which is inherent to placing the sequence encoding the fluorescent protein in 5' with respect to the Fokl catalytic domain.

In a particular embodiment, the nucleic acid molecule constitutes a bicistronic gene which in the 5'---3' transcription direction encodes at least one fusion protein according to the preceding features and a protein identifying transcription, preferably, a fluorescent protein.

In another particular embodiment, the nucleic acid molecule constitutes a tricistronic gene which in the 5'---3' transcription direction encodes at least one first TALE protein, a Fokl catalytic domain wherein at its C-terminal end it comprises a peptide comprising between 18 and 23 amino acids, preferably a 2A-type, a second TALE protein, a Fokl-type catalytic domain wherein at its C-terminal end it comprises a second peptide comprising between 18 and 23 amino acids, preferably a 2A-type, more preferably T2A-type, and a protein identifying transcription, preferably a fluorescent protein.

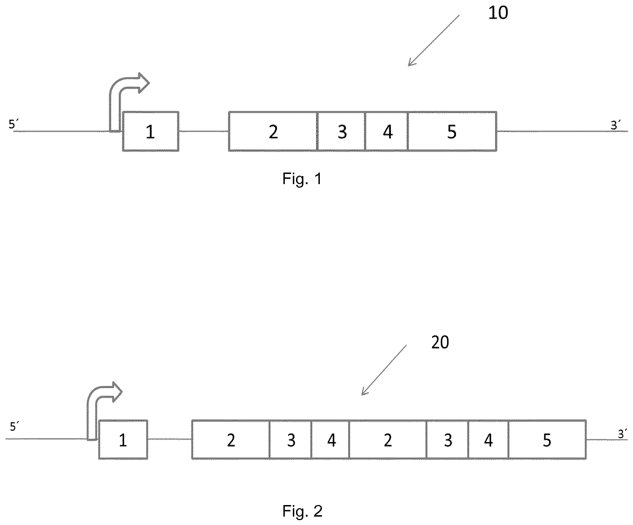

Figure 1 shows an example of a bicistronic gene 10 comprising an embodiment of the nucleic acid molecule of the invention expressing a fusion protein 100 and a fluorescent protein 50, said bicistronic gene comprising in the 5'---3' transcription direction:
- a sequence 1 encoding a promoter;
- the nucleic acid sequence 2 encoding the DNA binding domain comprising a first segment encoding the N-terminal end 20 of a TALE protein and a nuclear localization signal, a second segment encoding the TALE 21-type DNA binding domain, and a third segment 22 encoding the C-terminal end of the TALE protein; and
- the nucleic acid sequence encoding the Fokl-type catalytic domain comprising a first segment 3 encoding the Fokl-type catalytic domain 30 itself followed by a segment 4 encoding the peptide 40 comprising between 18 and 23 amino acids, preferably 23 amino acids; and
- the nucleic acid sequence 5 encoding the fluorescent protein 50.

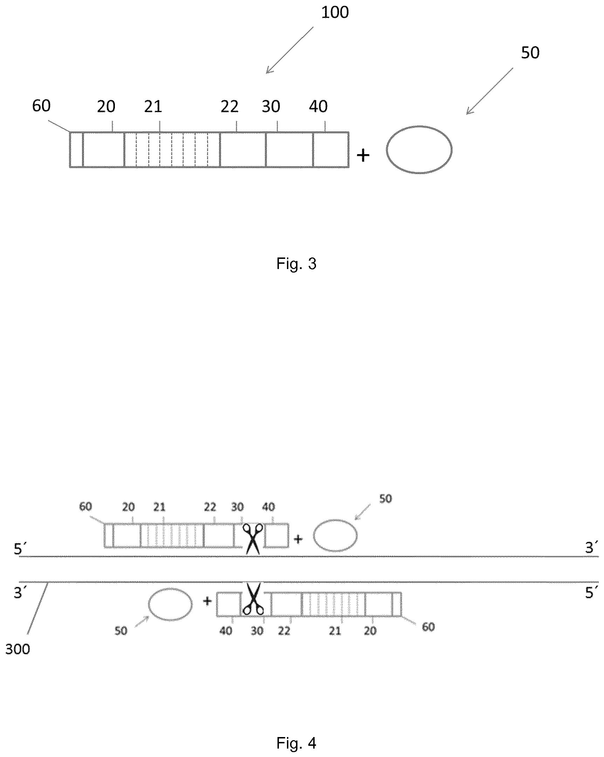

Figure 3 shows an example of the structure of the proteins produced from the bicistronic gene of Figure 1.

Figure 2 shows an example of a tricistronic gene comprising an embodiment of the nucleic acid molecule of the invention, in which a first fusion protein according to the invention 20, 21, 22, 30, 40, a second fusion protein 20, 21, 22, 30, 40 according to the invention and a fluorescent protein 50 are expressed from one and the same promoter 1.

An advantage of this arrangement of these polycistronic genes is that both the fusion protein and the fluorescent protein are expressed from one and the same promoter. This configuration minimizes the risk of selecting false positives which is inherent to placing the sequence encoding the fluorescent protein in 5' with respect to the Fokl catalytic domain.

When the nucleic acid molecule forming the monocistronic or polycistronic gene is a ribonucleic acid, said molecule comprises at the 5' end a structure referred to as CAP, which is known by the person skilled in the art. Generally, this structure provides stability to the messenger ribonucleic acid that may be transcribed from the gene.

When the nucleic acid molecule forming the monocistronic or polycistronic gene is a deoxyribonucleic acid molecule, the nucleotide sequences of the gene are adapted to the codon usage of the plant, animal or human organism, in which they are used.

Another aspect of the invention relates to a fusion protein comprising in the N-terminus to C-terminus direction at least one DNA binding domain and at least one Fokl-type catalytic domain, wherein said Fokl-type catalytic domain has a peptide comprising between 18 and 23 amino acids bound to its C-terminal end, preferably 21 amino acids.

In a particular embodiment, the DNA binding domain is a TALE-type domain.

In a particular embodiment, the peptide comprising between 18 and 23 amino acids is a 2A peptide, preferably a T2A-type peptide.

In a preferred embodiment, the T2A peptide comprises an amino acid sequence SEQ ID NO: 1.

In a particular embodiment, the fusion protein comprises in the N-terminus to C-terminus direction at least:
- one first segment consisting of between 50 and 200 amino acids long corresponding to the N-terminal end of a TALE protein;
- a TALE-type binding domain providing a target nucleotide sequence-specific binding;
- a segment consisting of between 20 and 100 amino acids long corresponding to the C-terminal end of a TALE protein; and
- a Fokl-type catalytic domain wherein at its C-terminal end it comprises a peptide consisting of between 18 and 23 amino acids, preferably 21 amino acids.

Said modified Fokl-type catalytic domain can be in its wild-type homodimer conformation or in its obligate heterodimer conformation.

Another aspect of the invention relates to a method for modifying the genetic material of a cell, comprising the steps of:
(i) providing a cell containing a target DNA nucleotide sequence; and
(ii) introducing in said cell at least one nucleic acid molecule according to any of the preceding features, or at least one nucleic acid molecule encoding at least one fusion protein according to any of the preceding features, and inducing expression of said at least one nucleic acid molecule; or
(iii) introducing in said cell at least one fusion protein according to any of the preceding features;
such that the binding domain recognizes the target nucleotide sequence and the Fokl-type catalytic domain can cut at a nucleotide sequence adjacent to the target nucleotide sequence.

In the context of the invention, the cell can be a plant cell or a mammalian human or animal cell.

In one embodiment, the cell is of a plant.

In another embodiment, the cell is a mammalian animal cell.

In another embodiment, the cell is a mammalian human cell. Examples of a human cell, without limitation, are lymphocytes, hematopoietic stem cells, fibroblasts from a biopsy or induced pluripotent stem cells.

The target nucleotide sequence can be in any cell type, tissue or eukaryotic organism.

In the context of the invention, adjacent is understood as inside the target sequence or at a distance of between 5 and 20 base pairs in the 5'----3' direction of the target sequence.

In a particular embodiment, at least two nucleic acid molecules are introduced in the cell, each molecule expressing a fusion protein of the invention. These at least two fusion proteins are complementary.

In another particular embodiment, at least two fusion proteins according to the invention are introduced in the cell. These at least two fusion proteins are complementary.

In the context of the invention, complementary is understood as at least each of the proteins recognizing a target sequence in a specific region of the genome of the cell, separated by between 5 and 20 base pairs of the one recognized by the other protein. The mechanism of action of the two complementary fusion proteins is known by the person skilled in the art as depicted in Figure 4:
- the binding domain 21 of a first fusion protein recognizes a first target nucleotide sequence to which it is bound, and the binding domain 21 of a second fusion protein recognizes a second target nucleotide sequence to which it is bound;
- the Fokl-type catalytic domains 30 of each fusion protein form a dimer between one another; and
- the dimer cuts at a nucleotide sequence between the target nucleotide sequences recognized by the binding domain of each fusion protein.

In the context of the invention, the Fokl catalytic domain can be in its wild-type homodimer conformation or in its obligate heterodimer conformation.

In a preferred embodiment, the nucleic acid molecule or nucleic acid molecules that are introduced in the cell comprise in the 5'---3' transcription direction at least:
- one nucleic acid sequence encoding a TALE-type DNA binding domain; and
- one nucleic acid sequence encoding a Fokl-type catalytic domain, wherein at its 3' end it comprises a nucleic acid sequence encoding a 2A-type peptide, preferably a T2A-type peptide, comprising an amino acid sequence SEQ ID NO: 1.

Additionally, the nucleic acid molecule of this preferred embodiment, in the 5'---3' direction after the 2A-type peptide, comprises a nucleic acid sequence encoding a protein identifying transcription, preferably a fluorescent protein. This confers additional advantages to the method since it allows identifying and selecting said cell.

In this preferred embodiment, the nucleic acid molecule, preferably nucleic acid, can form a polycistronic gene, preferably a bicistronic gene or a tricistronic gene as previously described.

In a particular embodiment, at least two nucleic acid molecules, preferably, two bicistronic genes are introduced in the cell. In this embodiment, preferably each bicistronic gene expresses a fusion protein 100 and a fluorescent protein 50 with a different emission spectrum and/or an emission spectrum that is distinguishable between both fluorescent proteins. This feature has an additional advantage, since it allows identifying and selecting by means of fluorescence the cells in which one, both or none of the fusion proteins encoded by the bicistronic genes is transcribed. The fusion proteins obtained from transcription of these two bicistronic genes are complementary. The identification and selection by fluorescence allows enriching a cell population genetically modified according to the method of the invention.

In a particular embodiment, the cells modified according to the method of the invention are identified and selected based on the fluorescence emitted by said cells. In another embodiment, these modified cells selected are amplified in culture and introduced back into at least two nucleic acid molecules described above. The cells modified in this second phase are identified and selected based on the fluorescence emitted by said cells. When the nucleic acid molecule introduced in the cell is the tricistronic gene described above, only one fluorescence will be obtained. In another particular embodiment, at least one tricistronic gene is introduced in the cell. In this embodiment, the tricistronic gene expresses two complementary fusion proteins and a fluorescent protein. This embodiment allows identifying by means of fluorescence those cells in which the two fusion proteins have been transcribed or expressed.

In any of the preceding cases, at least one tricistronic gene encoding two fusion proteins according to the invention complementary to one another and a single fluorescent protein could be introduced.

The nucleic acid molecule or nucleic acid molecules of the invention can be introduced by the different mechanisms known by the person skilled in the art. The nucleic acid molecule of the invention can be introduced in the cell by means of a viral or non-viral delivery system. Non-limiting examples of viral delivery systems comprise DNA viruses, RNA viruses, retroviral vectors, lentiviral vectors, adenoviruses, pox viruses, herpes viruses and/or adeno-associated viruses. Non-limiting examples of non-viral delivery comprise nucleofection, electroporation, lipofection, microinjection, biolistic delivery, virosomes, liposomes, immunoliposomes, niosomes, nucleic acid conjugates with polycations or lipids, naked DNA or DNA associated with a nanoparticle, artificial virions, agents inducing DNA uptake by the cell and sonoporation.

In a particular embodiment, the nucleic acid molecule is introduced in the form of a plasmid by means of nucleofection.

In another particular embodiment, the nucleic acid molecule is delivered by means of a lentiviral vector.

In another particular embodiment, the nucleic acid molecule is delivered by means of an adenovirus. Non-limiting examples of other viruses are adeno-associated viruses.

In one embodiment, the nucleic acid molecule or nucleic acid molecules expressing the fusion protein of the invention are delivered directly to the organism or patient (*in vivo*).

In another embodiment, the nucleic acid molecule or nucleic acid molecules expressing the fusion protein of the invention are delivered to cells in culture (*in vitro*), and once the cell is modified they are introduced in an organism or the patient (*ex vivo*).

In one embodiment, the cells in culture are cells obtained from the patient to be treated or from the organism to be genetically modified according to the method of the invention.

The nucleic acid molecules or the fusion proteins of the invention are used advantageously:
- for replacing a genome sequence with a non-identical homologous sequence (i.e., the homologous recombination);
- for deleting a genome sequence by means of cutting DNA at one or more sites in the genome, followed by eliminating the sequence located between the cutting points and their binding thereof;
- for introducing an exogenous sequence at a specific point of the genome where it previously was not located;
- for detecting the cell factors facilitating homologous recombination; and/or
- for replacing a wild-type sequence with a mutant sequence, or for converting one allele into a different allele.

The modification of genetic material is therefore applicable for the targeted genetic modification of a cell (deletion or insertion), for genome editing in its different forms, for generating genetically modified animals or plants, and for gene therapy.

Any pathology or disease in plants, animals and humans dependent on or mediated by a particular genome sequence, in any way, can be treated, corrected or alleviated using the methods and compositions described herein.

By way of example, the target nucleotide sequence is in a gene related to the treatment of acquired immunodeficiency disorders, cancer immunotherapy, lysosomal storage diseases (for example, Gaucher disease, GM1, Fabry disease and Tay-Sachs disease), mucopolysaccharidosis (for example, Hunter syndrome, Hurler syndrome), and hemoglobinopathies (for example, sickle cell, HbC, thalassemia and hemophilias).

The methods and compositions described herein also allow the treatment of infections (viral or bacterial) in a host, for example, by means of the blocking or inactivation of the expression of viral or bacterial receptors, thereby preventing infection and/or spread in a host organism. In a particular embodiment, the target nucleotide sequence is in the human CCR5 gene.

The human CCR5 gene encodes for the main co-receptor used by the human immunodeficiency virus (HIV), which causes AIDS, to penetrate in T cells. It has been found that eliminating the CCR5 co-receptor prevents entry of the virus in T cells, and therefore the progression of the infection and the development of the disease. In a particular embodiment, the molecule of the invention or the fusion proteins of the invention give rise to the genetic modification of the CCR5 gene giving rise to the inactivation of the CCR5 co-receptor in primary lymphocytes of AIDS patients. The genetic modification occurs in the section of DNA between the target nucleotide sequences of the human CCR5 gene recognized by the binding domains of at least two fusion proteins that are complementary to one another, the nucleotide target sequences preferably being SEQ ID NO: 2 and SEQ ID NO: 3.

In a particular embodiment, at least two nucleic acid molecules, preferably two complementary bicistronic genes of the invention, for example according to Figure 1, specific for the CCR5 gene, are introduced in the T cells in a first inactivation phase. The transcription of the genes produces two complementary TALEN-type fusion proteins and two fluorescent proteins, preferably mCherry and EGFP. The TALEN proteins will recognize their respective target sequences, preferably SEQ ID NO: 2 and SEQ ID NO: 3, located at such a distance that allows the two Fokl catalytic domains to dimerize and cut the double-strand of DNA. At the same time, the red and green fluorescences emitted by the respective fluorescent proteins allow identifying those cells in which the two bicistronic genes have been transcribed. The double fluorescence allows isolating them by means of fluorescence activation cell sorting (FACS) and thereby obtaining a cell population in which virtually 100% has at least one of the two CCR5 alleles inactivated. The T cells thus obtained can be subjected to a second inactivation phase. To that end, once they are isolated and amplified in culture, at least two complementary bicistronic genes of the invention as previously described are introduced again into the cells, therefore modifying the copy of the CCR5 gene that had not yet been modified. Both the T cells produced in the first phase, which will for the most part be heterozygotic for mutation of the CCR5 gene, and those produced in the second phase, which will for the most part be homozygotic for mutation of the CCR5 gene, are administered for therapeutic purposes to the original donor. The T cells produced are partially or completely resistant to infection by HIV, depending on whether they are homozygotic or heterozygotic for mutation of the CCR5 gene. The protection provided by the modified T cells will have a limited duration, so in order to extend it over time periodic administrations of modified T cells are performed.

To achieve permanent protection against HIV, hematopoietic stem cells in which the CCR5 gene was previously modified by means of the use of the molecule of the invention or the fusion protein of the invention or the method of the invention are transplanted in the patient. The method of inactivation will be the same as the one described for the case of T cells. The hematopoietic stem cells are previously obtained from the patient, or they could be produced *in vitro* from iPS cells derived from differentiated cells of the patient. Modification of the CCR5 gene can also be done in the iPS cells according to the process described above. In any of the previous cases, the method of modification of the CCR5 gene could also carried out by means of using a single tricistronic gene encoding two complementary fusion proteins according to the invention specific for the CCR5 gene and a single fluorescent protein.

In another particular embodiment, the target nucleotide sequence is in a gene related to a genetic disease, preferably monogenic.

By way of non-limiting example, genetic diseases include, but are not limited to, prolidase deficiency, sialidosis, galactosialidosis, α mannosidosis, β mannosidosis, aspartylglucosaminuria, fucosidosis, Schindler disease, metachromatic leukodystrophy, multiple sulfatase deficiency, globoid cell leukodystrophy, Pompe disease, Farber lipogranulomatosis, Wolman disease and cholesteryl ester storage disease, pycnodysostosis, ceroid lipofuscinosis, cystinosis, Salla disease, mucolipidosis III or IV, Danon disease, ceroid lipofuscinosis 6 and 8, Chediak Higashi syndrome, Griscelli syndrome type 1, 2 and 3, Hermansky Pudlak syndrome 2, X-linked retinoschisis, Stargardt disease, choroideremia, retinitis pigmentosa 1-57, achondroplasia, achromatopsia, acid maltase deficiency, adenosine deaminase deficiency (OMIM No. 102700), adrenoleukodystrophy, Aicardi syndrome, alpha-1 antitrypsin, alpha-thalassemia, androgen insensitivity syndrome, Apert syndrome, arrhythmogenic right ventricle, dysplasia, ataxia telangiectasia, Barth syndrome, beta-thalassemia, Bean syndrome, Canavan disease, chronic granulomatous diseases (CGD), Cri-du-Chat syndrome, cystic fibrosis, Dercum's disease, ectodermal dysplasia, Fanconi anemia, fibrodysplasia ossificans progressiva, fragile X syndrome, galactosemia, Gaucher disease, generalized gangliosidosis (for example, GM1), hemochromatosis, mutation of the hemoglobin C at codon 6 of beta-globin (HBC), hemophilia, Huntington's disease, Hurler syndrome, hypophosphatasia, Klinefelter syndrome, Krabbes disease, Langer-Giedion syndrome, leukocyte adhesion deficiency (LAD, OMIM No. 116920), leukodystrophy, long QT syndrome, Marfan syndrome, Moebius syndrome, mucopolysaccharidosis (MPS), Nail-patella syndrome, nephrogenic diabetes insipidus, neurofibromatosis, Niemann-Pick disease, osteogenesis imperfecta, the porphyria, Prader-Willi syndrome, progeria, Proteus syndrome, retinoblastoma, Rett syndrome, Rubinstein-Taybi syndrome, Sanfilippo syndrome, severe combined immunodeficiency (SCID), Shwachman syndrome, sickle cell disease (sickle cell anemia), Smith-Magenis syndrome, Stickler syndrome, Tay-Sachs disease, thrombocytopenia-absent radius (TAR), Down syndrome, Treacher Collins syndrome, trisomy, tuberous sclerosis, Turner lymphoproliferative syndrome, urea cycle disorder, von Hippel-Lindau disease, Waardenburg syndrome, Williams syndrome, Wilson disease, and X-linked Wiskott-Aldrich syndrome (XLP, OMIM No. 308240).

Therefore, another aspect of the invention relates to the use of the nucleic acid molecule or the fusion protein or the cell modified according to the method of the invention for use as a drug or for use in the treatment of the diseases mentioned above.

Another aspect of the invention relates to the use of the nucleic acid molecule of the invention or the fusion protein of the invention or the cell genetically modified according to the method of the invention, for preparing a drug or a therapeutic composition for the treatment or prevention of a disease. In a particular embodiment, said drug or said therapeutic composition is used for the treatment or prevention of AIDS.

In another particular embodiment, said drug or said therapeutic composition is used for the treatment or prevention of genetic disorders, preferably monogenic genetic disorders.

Another aspect of the invention relates to a method for the treatment or prevention of one of the diseases mentioned above, comprising the administration of a therapeutically effective amount of the nucleic acid molecules or of the fusion protein defined above, or of the cell genetically modified according to the method of the invention, together with pharmaceutically acceptable carriers or excipients, in a subject in need of said treatment and/or prevention, including a human.

The term "prevention or treatment" in the context of the invention means the administration of the nucleic acid molecules or the fusion proteins according to the invention for preserving the health of a patient who suffers from or is at risk of suffering from one of the diseases described above. Said terms also include the administration of the nucleic acid molecules or the fusion proteins according to the invention for preventing, improving, alleviating or eliminating one or more symptoms associated with the disease. The term "improving" in the context of this invention is understood to mean any improvement in the situation of the treated patient, whether subjectively (sensation of or in the patient) or objectively (measured parameters).

The nucleic acid molecules or the proteins or the modified cells of the present invention can be part of a therapeutic composition. Said therapeutic compositions include any solid, semi-solid or liquid composition.

The therapeutic composition of the invention comprises the nucleic acid molecule or the fusion protein or the cell modified according to the invention together with pharmaceutically acceptable carriers, excipients or vectors, in a subject in need of said treatment and/or prevention, including a human. The person skilled in the art can determine which additional components can be used and if they are required, many of them being commonly used in therapeutic compositions.

In the context of this invention, the expression "therapeutically effective amount" refers to the amount of composition which, once administered, is enough to prevent or treat one or more symptoms derived from the disease. The particular dose administered according to the present invention will be determined according to the particular circumstances surrounding the case, including the administered compound, the administration route, the particular condition being treated and similar considerations.

The expression "pharmaceutically acceptable carriers or excipients" refers to pharmaceutically acceptable materials, compositions or carriers. Each component must be pharmaceutically acceptable in the sense that they are compatible with the other ingredients of the pharmaceutical composition. It must also be suitable for use in contact with human and animal tissues or organs without excessive toxicity, irritation, allergic response, immunogenicity or other problems or complications consistent with a reasonable risk/benefit ratio.

Several illustrative examples showing the features and advantages of the invention are described below. Nevertheless, they must not be interpreted as being limiting of the object of the invention as defined in the claims.

### EXAMPLES

### Example 1: Preparation of a bicistronic nucleic acid molecule according to the invention

The new bicistronic genes have been constructed with the fluorescence protein bound by means of a 2A element to the C-terminal end of the Fokl monomer. Binding was done by means of successive PCR amplifications of the fragments to be assembled. Two fragments were amplified in a first series of reactions. The first one consisted of the 3' part of the region encoding for the Fokl catalytic domain and the 2A fragment. The second one had in its 5' part an area overlapping with the 3' end of the former, followed by the sequence encoding for the corresponding fluorescent protein. In the second amplification reaction, the two products of the first series were mixed with the primers corresponding to the 5' end of the first reaction and to the 3' end of the second reaction. The PCR reaction performed with this mixture gave rise to a DNA fragment that could be bound by means of classic molecular genetics methods to the classic structure of a TALEN, replacing the 3' fragment of the Fokl domain described in the prior art with another one containing the addition of 21 amino acids and the corresponding fluorescent protein.

### Example 2: Cutting efficacy of a fusion protein according to the invention

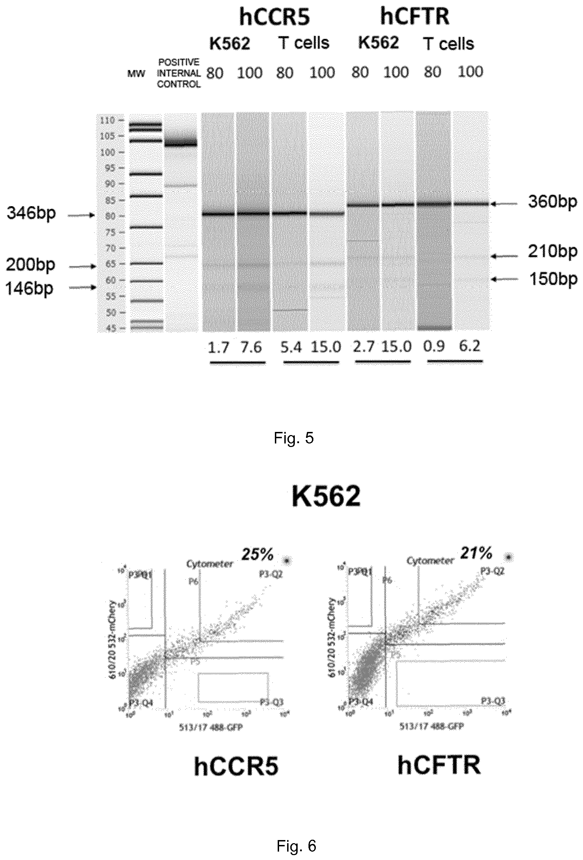

The cutting efficacies of two complementary pairs of fusion proteins 100 obtained according to Example 1 with respect to a complementary pair of a TALEN 80 of the prior art were compared. The proteins produced according to Example 1 have the fragment consisting of 21 amino acids with sequence SEQ ID NO: 1 added to the C-terminal end of the Fokl catalytic domain. The proteins 80 lack those 21 amino acids. The two complementary pairs of proteins are specific for the regions proximal to Δ32 in the CCR5 gene and ΔF508 in the CFTR gene, respectively, both being human genes, in two cell types: K562 and primary T cells. The genes encoding for both proteins were introduced in plasmid form in the cells by means of nucleofection (LONZA). The Surveyor® method known by the person skilled in the art was used to measure cutting efficacy. The results were quantified by means of virtual electrophoresis and densitometry performed with an Agilent Technologies Bioanalyzer 2100 molecular analyzer. The same amount of cells and the same amount of the proteins 100 and 80 were used for each experiment. Both proteins recognize the same target sequences within each gene. Cutting efficacy is measured with the Surveyor method by the relative abundance of digestion products compared with the uncut target sequence (346 bp for CCR5 and 360 bp for CFTR). Figure 5 shows the efficacy results obtained. The obtained values (as percentages of the unmodified allele) are indicated below each column. The arrows indicate the position and the size of unmodified alleles (bands corresponding to 346 bp and 360 bp) and of the corresponding digestion products (200 bp, 146 bp, 210 bp and 150 bp). MW: molecular weight marker. Surveyor® positive control: internal operation control of the kit. The numerical scale on the left consists of arbitrary shifting values used by the apparatus where the measurement was taken (Bioanalyzer).

Based on the obtained results, it is concluded that the fusion proteins of the invention are more effective than the proteins of the prior art.

### Example 3: Identification and enrichment of a cell population subjected to the method of genetic modification according to the invention

The results of two experimental series performed with K562 cells are presented. A different gene target has been inactivated in each series. In one case, the gene target was CCR5 and in other CFTR. The corresponding cells were transfected in each experiment with a pair of bicistronic genes (hereinafter referred to as TALEN-F1 and TALEN-F2), each bicistronic gene encoding a fusion protein 100 and a fluorescent protein 50. The fusion proteins obtained from the transcription of the two types of bicistronic gene are complementary (specific for the regions proximal to Δ32 in the CCR5 gene and ΔF508 in the gene CFTR, respectively), whereas the fluorescent proteins obtained with each type of gene have a different emission spectrum and/or an emission spectrum that is distinguishable between both fluorescent proteins. One of the genes encodes a fluorescent protein, mCherry, and the other gene encodes the EGFP protein. The identification and selection by fluorescence allows enriching a cell population genetically modified according to the method of the invention, and the obtained results demonstrate this. In Figure 6, the scatter plots depict the distribution of cells according to the intensity of the expression of the two fluorescent proteins obtained by the cell sorter. The emission corresponding to GFP is represented on the x-axis, and the emission corresponding to mCherry is represented on the y-axis. The parameters of the cell sorter have been adjusted so that the cells only expressing one of the two fluorescent proteins are grouped next to the double negatives in the lower left quadrant. The upper right quadrant contains the cells expressing the two fluorescent proteins with the highest intensity. The percentages correspond to the percentage of cells expressing the two fluorescent proteins compared to the total cells of each experiment.

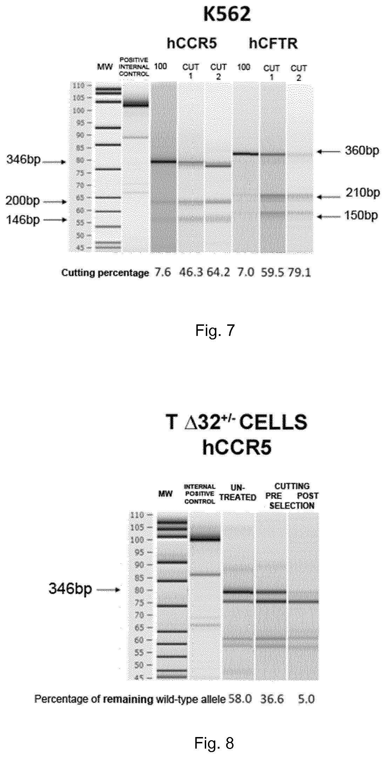

The nuclease or cutting activity of the fusion proteins of the invention was determined in a second phase by means of the aforementioned Surveyor method®. To that end, DNA was extracted from isolated cells expressing the two fluorescent proteins. Figure 7 reflects the results obtained with the human CCR5 and CFTR genes in K562 cells. The same amount of cells and bicistronic genes was used in each case and the fusion protein cutting percentages were compared in three situations:
- Transfection with TALEN-F1 and TALEN-F2 and without performing selection by fluorescence (column 100).
- First transfection with TALEN-F1 and TALEN-F2 and selection by fluorescence (cut column 1).
- Second transfection with TALEN-F1 and TALEN-F2 and selection by fluorescence (cut column 2).

After the first transfection and selection cycle (cut 1), it can be seen how the cutting percentages are around 50%. This means that about half of the copies of the gene present in each DNA sample has been cut by the fusion proteins expressed by TALEN-F1 and TALEN-F2. Since all the cells contain two copies of each gene, the result can be interpreted as at least one copy of the gene having been modified in virtually all the selected cells. In cut 2, it is seen that the second transfection cycle produces an increase in cutting percentages. The Surveyor method® is not precise enough to quantitatively establish the percentage of homozygotic cuts, but it can be seen in Figure 7 how the band corresponding to the 346 bp wild-type allele in CCR5 and 360 bp wild-type allele in CFTR has disappeared, giving way to other bands of a different size. It can therefore be concluded that the method is effective for obtaining mutant homozygotic cells.

### Example 4: Identification and enrichment of T cells subjected to the method of genetic modification according to the invention

In order to check the efficiency of the method in the production of T cells with two null copies of the CCR5 gene, an experiment was conducted in which donor heterozygotic T cells were used for the Δ32 mutation. The T cells were nucleofected, according to the method of the invention, with a pair of bicistronic genes specific for the CCR5 gene, each bicistronic gene encoding a fusion protein 100 comprising SEQ ID NO: 1 and a fluorescent protein 50. The fusion proteins obtained from the transcription of the two types of bicistronic gene are complementary, one having a binding domain specific for SEQ ID NO: 2 and the one having a binding domain specific for SEQ ID NO: 3. One of the genes encodes a fluorescent protein, mCherry, and the other gene encodes the protein EGFP. The Surveyor method® mentioned above was used to determine the nuclease or cutting activity of the fusion proteins of the invention, and at the same time to determine the frequency of the wild-type allele of the CCR5 gene in the treated cell population. DNA samples from the original, untreated population, from the population immediately after nucleofection, but without having been selected, and from the population obtained once the lymphocytes that expressed the two fluorescent proteins were selected by cell sorting, were compared. Figure 8 shows the obtained results. It can be seen how the frequency of the wild-type allele decreases with treatment, until virtually disappearing in the cell population selected by fluorescence.

## Claims

1. A nucleic acid molecule encoding a fusion protein, the nucleic acid molecule comprising in the 5'---3' transcription direction at least:
(a) one nucleic acid sequence encoding a DNA binding domain; and
(b) one nucleic acid sequence encoding a Fokl-type catalytic domain, wherein at its 3' end it comprises a nucleic acid sequence encoding a peptide comprising between 18 and 23 amino acids.

2. The nucleic acid molecule according to claim 1, wherein the peptide is a 2A-type peptide.

3. The nucleic acid molecule according to claim 2, wherein the DNA binding domain is a TALE-type domain.

4. The nucleic acid molecule according to claim 3, wherein in the 5'---3' transcription direction:
(a) the nucleic acid sequence encoding the DNA binding domain comprises a first segment consisting of between 150 and 600 base pairs, preferably between 470 and 550 base pairs, encoding the N-terminal end of a TALE protein and a nuclear localization signal, a second segment consisting of between 1100 and 2900 base pairs encoding the TALE-type DNA binding domain, and a third segment consisting of between 40 and 850 base pairs encoding the C-terminal end of the TALE protein; and
(b) the nucleic acid sequence encoding the Fokl-type catalytic domain comprises a first segment consisting of between 550 and 600 base pairs, preferably between 590 and 600 base pairs, encoding the Fokl-type catalytic domain followed by a segment consisting of between 50 and 70 base pairs, preferably 69 base pairs, encoding the peptide comprising between 18 and 23 amino acids, preferably 21 amino acids.

5. The nucleic acid molecule according to claim 4, wherein the peptide is a T2A-type peptide and comprises a sequence SEQ ID NO: 1.

6. The nucleic acid molecule according to any of the preceding claims, wherein in the 5'---3' transcription direction after the nucleic acid sequence encoding the peptide comprising between 18 and 23 amino acids, it comprises a nucleic acid sequence encoding a protein identifying transcription.

7. The nucleic acid molecule according to claim 6, wherein the nucleic acid sequence encoding the protein identifying transcription comprises between 700 and 1000 base pairs, and the protein identifying transcription is a fluorescent protein.

8. The nucleic acid molecule according to any of the preceding claims, wherein the nucleic acid is a deoxyribonucleic acid molecule or a ribonucleic acid molecule.

9. The nucleic acid molecule according to any of the preceding claims, wherein said molecule forms a monocistronic gene or a polycistronic gene.

10. A fusion protein comprising in the N-terminus to C-terminus direction at least one DNA binding domain and at least one Fokl-type catalytic domain, wherein said Fokl-type catalytic domain has a peptide comprising between 18 and 23 amino acids bound to its C-terminal end.

11. The fusion protein according to claim 10, wherein the DNA binding domain is a TALE-type domain.

12. The fusion protein according to claim 10 or 11, wherein the peptide is a 2A peptide, preferably a T2A-type peptide.

13. The fusion protein according to any of claims 10 to 12, wherein the peptide comprises a sequence SEQ ID NO: 1.

14. A method for modifying the genetic material of a cell, comprising the steps of:
(i) providing a cell containing a target DNA nucleotide sequence; and
(ii) introducing in said cell at least one nucleic acid molecule according to any of claims 1 to 9, or at least one nucleic acid molecule encoding at least one fusion protein according to any of claims 10 to 13, and inducing expression of said at least one nucleic acid molecule; or
(iii) introducing in said cell at least one fusion protein according to any of claims 10 to 13,
such that the binding domain recognizes the target nucleotide sequence and the Fokl-type catalytic domain can cut at a nucleotide sequence adjacent to the target nucleotide sequence.

15. The method according to claim 14, wherein at least one nucleic acid molecule according to claim 6 or 7 is introduced, wherein the nucleic acid molecule comprises a nucleic acid sequence encoding a fluorescent protein.

16. The method according to claim 14, wherein at least two nucleic acid molecules according to claim 6 or 7 are introduced, wherein each nucleic acid molecule comprises a nucleic acid sequence encoding a fluorescent protein, each fluorescent protein having a different emission spectrum.

17. The method according to claim 15 or 16, wherein the cells in which the genetic material has been modified are identified and selected based on the fluorescence emitted by said cells.

18. The method according to claim 17, wherein the selected cells are amplified in culture and the nucleic acid molecule or nucleic acid molecules are introduced again into cells, and wherein the cells in which the genetic material has been modified again are identified and selected based on the fluorescence emitted by said cells.

19. The method according to any of claims 14 to 18, wherein the genetic material is introduced in said cell by a viral vector system and/or by a non-viral vector system.

20. The method according to any of claims 14 to 19, wherein the target nucleotide sequence is in the human CCR5 gene, which encodes a membrane co-receptor present in T cells of the human immunodeficiency virus (HIV).

21. The method according to claim 20, wherein the modification of the genetic material of the T cell gives rise to the elimination and/or inactivation of the membrane co-receptor.

22. A therapeutic composition comprising at least one nucleic acid molecule according to any of claims 1 to 9, at least one fusion protein according to any of claims 10 to 13, or at least one genetically cell modified according to any of claims 14 to 21.

23. The therapeutic composition according to claim 22 for the treatment of genetic disorders.

24. The therapeutic composition according to claim 22 for use in the treatment of AIDS.
